# FASCICULE DE BREVET EUROPEEN

(11) **EP 0 759 030 B1**
(45) Date de publication et mention de la délivrance du brevet: **09.09.1998**
(21) Numéro de dépôt: 95919483.8
(22) Date de dépôt: 05.05.1995
(51) Int. Cl.: C07K 5/072

(54) **N- N-(3,3-DIMETHYLBUTYL)-L-$g(a)-ASPARTYL]-L-HEXAHYDROPHENYLALANINE 1-METHYL ESTER UTILE COMME AGENT EDULCORANT, SON PROCEDE DE PREPARATION**
N-[N-(3,3-DIMETHYLBUTYL)-L-ALPHA-ASPARTYL]-L-HEXAHYDROPHENYLALANINE 1-METHYLESTER, ALS SÜSSSTOFF VERWENDBAR UND DEREN HERSTELLUNG
N- N-(3,3-DIMETHYLBUTYL)-L-$g(a)-ASPARTYL]-L-HEXAHYDROPHENYLALANINE 1-METHYL ESTER USEFUL AS A SWEETENING AGENT AND METHOD OF PREPARATION

(30) Priorité: 09.05.1994 FR 9405675
(43) Date de publication de la demande: 26.02.1997
(73) Titulaire: NOFRE, Claude, F-69003 Lyon (FR); TINTI, Jean-Marie, F-69680 Chassieu (FR)
(72) Inventeur: NOFRE, Claude, F-69003 Lyon (FR); TINTI, Jean-Marie, F-69680 Chassieu (FR)
(74) Mandataire: Hubert, Philippe
(86) Numéro de dépôt international: FR9500588
(87) Numéro de publication internationale: WO9530688

(56) Documents cités:
- AT-A- 294 035

## Description

La présente invention concerne un nouveau composé utile comme agent édulcorant, ainsi que son procédé de préparation.

Ce nouveau composé est particulièrement utile pour édulcorer des produits variés, en particulier les boissons, les aliments, les confiseries, les pâtisseries, les chewing-gums, les produits d'hygiène et les articles de toilette, ainsi que les produits cosmétiques, pharmaceutiques et vétérinaires.

On sait qu'un agent édulcorant, pour être utilisable à l'échelle industrielle, doit posséder, d'une part, un pouvoir sucrant intense permettant de limiter le coût d'utilisation, et, d'autre part, une stabilité satisfaisante, c'est-à-dire compatible avec les conditions d'utilisation.

Parmi les agents édulcorants actuellement commercialisés, un dérivé dipeptidique, le *N*-L-α-aspartyl-L-phénylalanine 1-méthyl ester, connu sous le nom d'aspartame, de formule suivante: 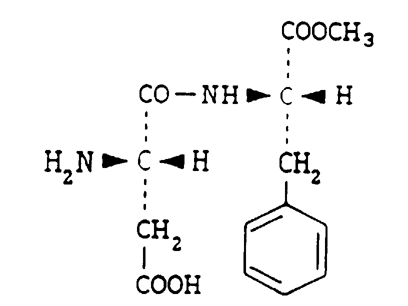 est aujourd'hui le plus utilisé (US 3,492,131). Le pouvoir sucrant de l'aspartame est relativement faible, puisqu'il est inférieur à 200 fois celui du saccharose sur une base pondérale. Malgré d'excellentes qualités organoleptiques, ce composé a pour principal inconvénient d'être un produit assez cher, en raison de sa faible intensité édulcorante, mais aussi d'avoir une assez basse stabilité dans certaines conditions d'utilisation des édulcorants, notamment en milieu neutre, ce qui limite ses champs d'applications industrielles.

En conséquence, il est apparu nécessaire pour l'industrie alimentaire de pouvoir disposer d'un nouvel agent édulcorant qui présenterait une activité édulcorante élevée, ceci afin de diminuer son prix de revient, et qui serait au moins aussi stable et même plus stable que l'aspartame, notamment en milieu neutre.

Dans un premier aspect, l'invention concerne un nouveau composé édulcorant, le *N*-[*N*-(3,3-diméthylbutyl)-L-α-aspartyl]-L-hexahydrophénylalanine 1-méthyl ester de formule :

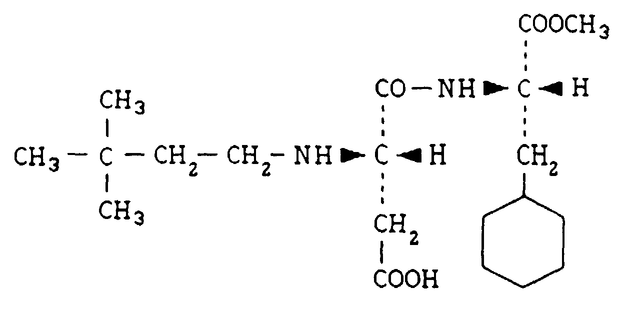

Ce nouveau composé édulcorant est donc un dérivé *N*-alkylé d'un composé hexahydrogéné de l'aspartame (hexahydroaspartame).

L'hexahydroaspartame, de formule : 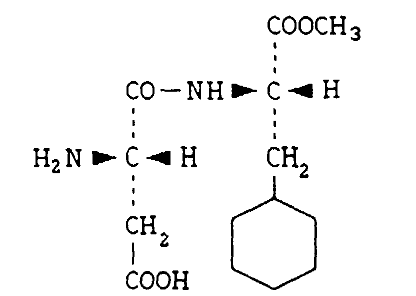 a déjà été décrit dans la littérature et notamment dans le brevet français FR 2.013.158. Son pouvoir sucrant, assez proche de celui de l'aspartame, est environ 225 fois celui du saccharose sur une base pondérale.

Il a été découvert, de façon tout à fait inattendue, que le composé de l'invention est, sur une base pondérale, environ 50 (cinquante) fois plus sucré que l'hexahydroaspartame, environ 60 (soixante) fois plus sucré que l'aspartame et 12 000 (douze mille) fois plus sucré que le saccharose (sucre de table).

Il a de plus été observé que la stabilité du composé de l'invention est plus élevée que celle de l'aspartame dans les conditions courantes d'utilisation pour les préparations alimentaires. Ceci est un avantage d'autant plus important que l'une des limites à l'utilisation de l'aspartame dans certaines préparations alimentaires provient de sa stabilité très faible en milieu proche de la neutralité, c'est-à-dire pour des pH voisins de 7, pH qui sont fréquemment rencontrés dans des produits tels que les produits laitiers, les pâtisseries ou autres préparations qui nécessitent une cuisson à haute température, les chewing-gums, les dentifrices.

Bien qu'obtenu directement à partir de l'aspartame, le composé de l'invention présente l'avantage de ne pas contenir de L-phénylalanine, le groupe phényle de cet amino acide se trouvant réduit en groupe cyclohexyle dans le composé de l'invention. De plus, du fait du pouvoir sucrant très élevé du composé de l'invention, son utilisation dans les produits alimentaires pourra se faire avec des concentrations 60 fois plus faibles que celles de l'aspartame. En conséquence, dans les produits alimentaires, la présence, souvent débattue, de certains constituants de l'aspartame, à savoir la présence de méthanol ou de L-phénylalanine, sera respectivement très fortement réduite ou supprimée par l'emploi du composé de la présente invention.

La présente demande qui vise à couvrir le composé de l'invention en tant qu'agent édulcorant couvre aussi les compositions édulcorantes incorporant à titre d'agent édulcorant le composé de l'invention ainsi que son utilisation pour édulcorer les produits variés rappelés en introduction.

L' agent édulcorant de la présente invention peut être ajouté à tout produit comestible dans lequel on désire apporter un goût sucré, à condition de l'ajouter en proportions suffisantes pour atteindre le niveau de sucrosité désiré. La concentration optimale d'utilisation de l'agent édulcorant dépendra de facteurs divers tels que, par exemple, les conditions de stockage et d'utilisation des produits, les constituants particuliers des produits et le niveau de sucrosité désiré. Toute personne qualifiée peut facilement déterminer la proportion optimale d'agent édulcorant qui doit être employée pour l'obtention d'un produit comestible en réalisant des analyses sensorielles de routine. L'agent édulcorant de la présente invention est, en général, ajouté aux produits comestibles dans des proportions allant de 0,5 mg à 50 mg d'agent édulcorant par kilogramme ou par litre de produit comestible. Les produits concentrés contiendront évidemment des quantités plus élevées d'agent édulcorant, et seront ensuite dilués suivant les intentions finales d'utilisation.

L'agent édulcorant de la présente invention peut être ajouté sous forme pure aux produits à édulcorer, mais il est généralement mélangé à un support ("carrier") ou à un agent de charge ("bulking agent") approprié en raison de son pouvoir sucrant élevé.

Avantageusement, les supports ou agents de charge appropriés sont choisis dans le groupe constitué par le polydextrose, l'amidon, les maltodextrines, la cellulose, la méthylcellulose, la carboxyméthylcellulose et autres dérivés de la cellulose, l'alginate de sodium, les pectines, les gommes, le lactose, le maltose, le glucose, la leucine, le glycérol, le mannitol, le sorbitol, le bicarbonate de sodium, les acides phosphorique, citrique, tartrique, fumarique, benzoïque, sorbique, propionique, et leurs sels de sodium, potassium et calcium, et leurs équivalents.

L'agent édulcorant de l'invention peut, dans un produit comestible, être employé comme unique agent édulcorant, ou en combinaison avec d'autres agents édulcorants tels que le saccharose, le sirop de maïs, le fructose, les dérivés ou analogues dipeptidiques sucrés (aspartame, alitame), la néohespéridine dihydrochalcone, l'isomaltulose hydrogéné, le stévioside, les sucres L, la glycyrrhizine, le xylitol, le sorbitol, le mannitol, l'acésulfame, la saccharine et ses sels de sodium, potassium, ammonium et calcium, l'acide cyclamique et ses sels de sodium, potassium et calcium, le sucralose, la monelline, la thaumatine, et leurs équivalents.

Dans un second aspect, l'invention concerne le procédé de préparation du composé précité, procédé qui est caractérisé en ce que l'on traite une solution d'aspartame et de 3,3-diméthylbutyraldéhyde à température ambiante par l'hydrogène à une pression relative de 3 bars (0,3 MPa) en présence d'un catalyseur à base de platine.

Dans une forme avantageuse de réalisation de ce procédé, le catalyseur précité est choisi dans le groupe comprenant le noir de platine et l'oxyde de platine.

Le suivi de l'état d'avancement de la réaction par prélèvement et évaluation du produit formé par chromatographie liquide haute performance (H.P.L.C.) permet alors à l'homme de l'art de déterminer aisément la durée d'hydrogénation appropriée aux conditions utilisées.

Dans une mise en oeuvre actuellement préférée de l'invention, la solution d'aspartame et de 3,3-diméthylbutyraldéhyde est une solution hydroalcoolique de pH 4,5-5 obtenue par mélange d'une solution d'acide acétique 0,1 M et de méthanol, la concentration d'aspartame dans le solvant hydroalcoolique étant comprise entre 50 et 60 g/L et la concentration de 3,3-diméthylbutyraldéhyde entre 20 et 30 g/L.

Dans un aspect avantageux de l'invention, le produit formé est purifié par précipitation et filtration après élimination sous vide de la partie alcoolique du solvant.

Le composé de l'invention peut aussi être obtenu à partir d'autres procédés. On peut notamment soumettre le *N*-L-α-aspartyl-L-hexahydrophénylalanine 1-méthyl ester à une N-alkylation réductive par le 3,3-diméthylbutyraldéhyde sous l'action du cyanoborohydrure de sodium ou de l'hydrogène en présence de catalyseurs (voir par exemple Ohfune *et al.,* Chem. Letters, 1984, pp. 441-444 et la revue de P. N. Rylander, "Catalytic Hydrogenation in Organic Syntheses", Academic Press, San Diego, 1993, pp. 165-174).

Le procédé de l'invention demeure cependant beaucoup plus avantageux en ce qu'il permet d'obtenir le nouveau composé édulcorant directement, et en une seule étape, à partir de l'aspartame. Ce procédé permet en effet de réaliser simultanément la *N*-alkylation réductive par le 3,3-diméthylbutyraldéhyde et l'hydrogénation du groupe phényle de l'aspartame en groupe cyclohexyle.

Cependant, la mise en oeuvre simultanée de la *N*-alkylation réductive et de l'hydrogénation du groupe phényle ne s'est avérée possible que pour un nombre restreint de catalyseurs et dans des conditions expérimentales très spécifiques qui seules permettent d'obtenir la haute pureté analytique nécessaire pour l'utilisation alimentaire du composé faisant l'objet de l'invention.

Il faut noter que l'utilisation de l'aspartame comme réactif de base pour préparer le composé de l'invention a nécessité la résolution de certaines difficultés liées aux propriétés de l'aspartame.

En effet, l'aspartame présente une solubilité relativement faible dans la plupart des solvants organiques; sa solubilité est généralement inférieure à quelques grammes par litre.

Par ailleurs, si la solubilité de l'aspartame est plus élevée dans les milieux aqueux, sa stabilité est cependant relativement faible dans ces milieux.

En outre, toute tentative d'élévation de la température dans le but d'améliorer la solubilité de l'aspartame aggrave ses processus de dégradation.

Le procédé selon l'invention permet l'utilisation de l'aspartame comme réactif de départ tout en respectant les contraintes de pureté que l'on peut exiger d'un composé dont la destination principale est l'usage alimentaire.

Il a été en effet constaté que la qualité du produit de l'invention dépend très étroitement des conditions expérimentales appliquées durant la mise en oeuvre du procédé. La nature du catalyseur, et dans une moindre mesure la durée et la pression d'hydrogénation, la nature du milieu réactionnel et son pH se sont ainsi révélés être des paramètres essentiels.

En résumé, la présente invention décrit un nouveau composé qui présente par rapport à l'aspartame un pouvoir édulcorant 60 fois plus élevé et une plus grande stabilité tant en milieu neutre qu'en milieu acide. De plus, ce nouveau composé est préparé directement et en une seule étape à partir de l'aspartame, avec un haut rendement et une grande pureté.

La présente invention sera plus complètement décrite à l'aide des exemples de préparation suivants, qui ne doivent pas être considérés comme limitatifs de la portée de l'invention.

### EXEMPLE 1

Dans un réacteur équipé d'une agitation permettant d'assurer un très bon transfert de l'hydrogène gazeux dans la phase liquide, on introduit sous agitation et dans l'ordre : 60 cm³ d'une solution aqueuse d'acide acétique 0,1 M, 1 g de noir de platine (Aldrich n° 20,591-5 : Platinum black), 2,55 g de 3,3-diméthylbutyraldéhyde, 30 cm³ de méthanol et 5 g d'aspartame.

Après avoir purgé le réacteur par un courant d'azote, le mélange est soumis à une hydrogénation à la pression relative de 3 bars (0,3 MPa) et à température ambiante. L'avancement de la réaction est contrôlé par prélèvement d'un échantillon brut et dosage du produit formé par chromatographie liquide haute performance (H.P.L.C.). La concentration en produit désiré est déterminée par comparaison avec une courbe d'étalonnage préalablement établie. Après 72 heures d'hydrogénation, on observe la formation de 97 % du produit attendu.

La réaction est alors interrompue en purgeant le réacteur par un courant d'azote et en séparant le catalyseur par filtration sur filtre fin (0,5 µm). Si besoin est, le filtrat est ajusté à pH 5 par addition de quelques gouttes d'une solution d'hydroxyde de sodium 1 N. Le méthanol est alors éliminé par évaporation sous vide, la température étant maintenue au-dessous de 40 °C. Un solide blanc précipite rapidement. Le mélange est encore agité quelques heures à température ambiante pour compléter la précipitation. Le produit est séparé par filtration, séché et lavé par environ 50 cm³ d'hexane. On obtient finalement 4,5 g de *N*-[N-(3,3-diméthylbutyl)-L-α-aspartyl]-L-hexahydrophénylalanine 1-méthyl ester (rendement 69 %) sous la forme d'une poudre blanche de grande pureté (supérieure à 98 % par H.P.L.C.) .

### EXEMPLE 2

En utilisant le même appareillage, le même solvant et les mêmes réactifs aux mêmes concentrations que ceux décrits dans l'exemple 1, mais en employant comme catalyseur 1 g d'oxyde de platine (produit Aldrich n° 20,603-2 : Platinum(IV) oxide, Adams' catalyst), en effectuant l'hydrogénation à la pression relative de 3 bars (0,3 MPa), toujours à température ambiante, la réaction est arrêtée après 72 heures (96 % de produit formé). Après purification par précipitation suivant le protocole décrit dans l'exemple 1, on obtient 4,5 g du produit attendu (rendement 69 %) sous forme de poudre blanche de très grande pureté (supérieure à 98 % par H.P.L.C.).

La pureté du composé préparé suivant le procédé de l'invention est contrôlée par les techniques classiques de chromatographie sur couche mince, spectrométrie en infrarouge, spectrométrie en ultraviolet, chromatographie liquide haute performance (H.P.L.C.), analyse thermique, pouvoir rotatoire, résonance magnétique nucléaire et analyse centésimale.

Les critères physiques obtenus pour le composé préparé selon l'invention sont donnés ci-après.
Formule moléculaire : C₂₀H₃₆N₂O₅.
Poids moléculaire : 384,51.
Teneur en eau (méthode de Karl Fischer) : 3 à 5 %.
Chromatographie sur couche mince : Gel de silice 60 F254 sur feuilles d'aluminium (Merck n°5554), éluant : butanol-acide acétique-eau (8:2:2), révélation à la ninhydrine : Rf : 0,62.
Spectre infrarouge (KBr) cm⁻¹ : 3514 (HOH), 3367, 3195 (NH), 2957, 2920, 2850 (CH), 1755 (COOCH₃), 1659 (CONH), 1620-1593 (COO⁻), 1450, 1388, 1199, 1177, 773.
Spectre ultraviolet : maximum à 222 nm.
Chromatographie liquide haute performance sur colonne Merck de type "Lichrospher 100 RP-18 endcapped", longueur 244 mm, diamètre 4 mm, éluant : acétate d'ammonium 65 mM - acétonitrile (50:50), débit : 1 ml/min, détecteur : réfractomètre, temps de rétention 4,9 min.
Analyse thermique différentielle de 40 à 350 °C à 10 °C/min : point de fusion 88 °C, pas de décomposition au-dessous de 200 °C.
Pouvoir rotatoire : [α]D₂₀ = - 40 ° ± 1,25 (c = 2, méthanol).
Spectre de résonance magnétique nucléaire (H, 200 MHz, DMSO-d6) 0,86 (s, 9 H), 1,12 à 1,62 (m, 12 H), 2,3 à 2,45 (m, 5 H), 2,9 (m, 2 H), 3,55 (m, 1 H), 3,61 (s, 3 H), 4,4 (m, 1 H), 8,5 (d, 1 H).
Analyse centésimale : trouvé (théorique pour 4,5 % d'eau) :
C 59,63 (59,78), H 9,15 (9,51), N 6,66 (6,97), O 23,35 (23,72).

Le pouvoir édulcorant du composé de la présente invention a été évalué par un groupe de huit personnes expérimentées. Pour cela, le composé, en solution aqueuse à des concentrations variables, est comparé, sur le plan gustatif, à une solution témoin de saccharose à 2 %, à 5 % ou à 10 %. Le pouvoir édulcorant du composé, testé par rapport au saccharose, correspond alors au rapport pondéral qui existe entre le composé et le saccharose à égale intensité édulcorante, c'est-à-dire quand les saveurs sucrées de la solution du composé testé et de la solution témoin de saccharose sont considérées, par une majorité de personnes, avoir la même intensité édulcorante.

Le pouvoir édulcorant du composé de l'invention correspond approximativement, sur une base pondérale, à 12 000 fois celui du saccharose par comparaison avec une solution de saccharose à 2 %, à 10 000 fois par comparaison avec une solution de saccharose à 5 % et à 5 000 fois par comparaison avec une solution de saccharose à 10 %.

La stabilité du composé de l'invention et de l'aspartame a été mesurée en dosant, par chromatographie liquide haute performance, la quantité de produit restant après un vieillissement accéléré par chauffage prolongé à 70 °C en milieu neutre (tampon phosphate à pH 7) ou en milieu acide (tampon phosphate à pH 3). La stabilité du composé ainsi testé est évaluée par sa demi-vie (temps correspondant à 50 % de dégradation).

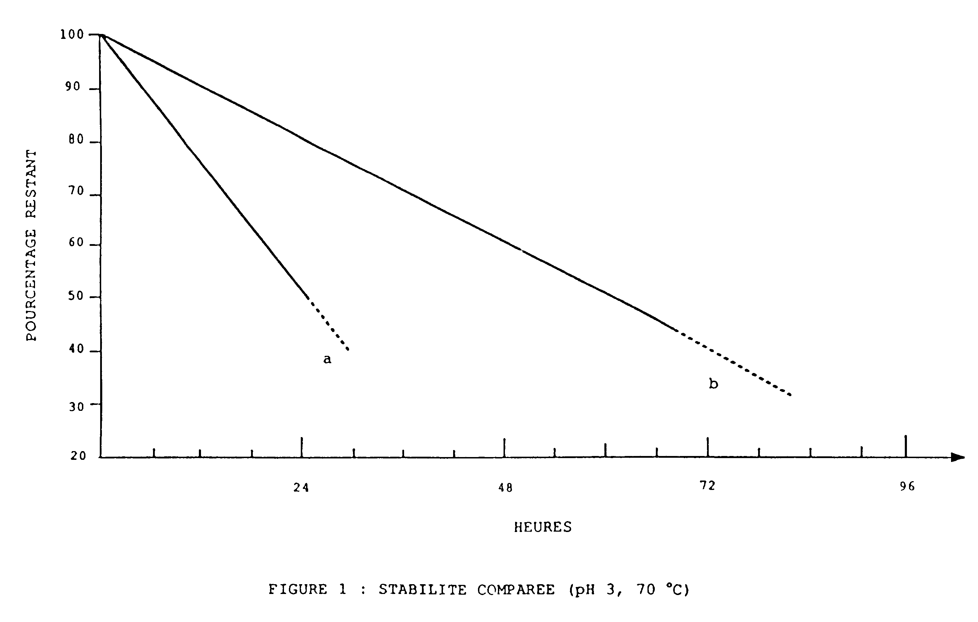

Dans la figure 1 annexée, est donné un diagramme comparatif des courbes de stabilité de l'aspartame (courbe a) par rapport au composé de l'invention (courbe b) en milieu acide à pH 3; le composé de l'invention présente une demi-vie d'environ 60 heures, alors que la demi-vie de l'aspartame dans les mêmes conditions n'est que de 24 heures, ce qui correspond, pour le composé selon l'invention, à une stabilité 2,5. fois plus élevée que celle de l'aspartame.

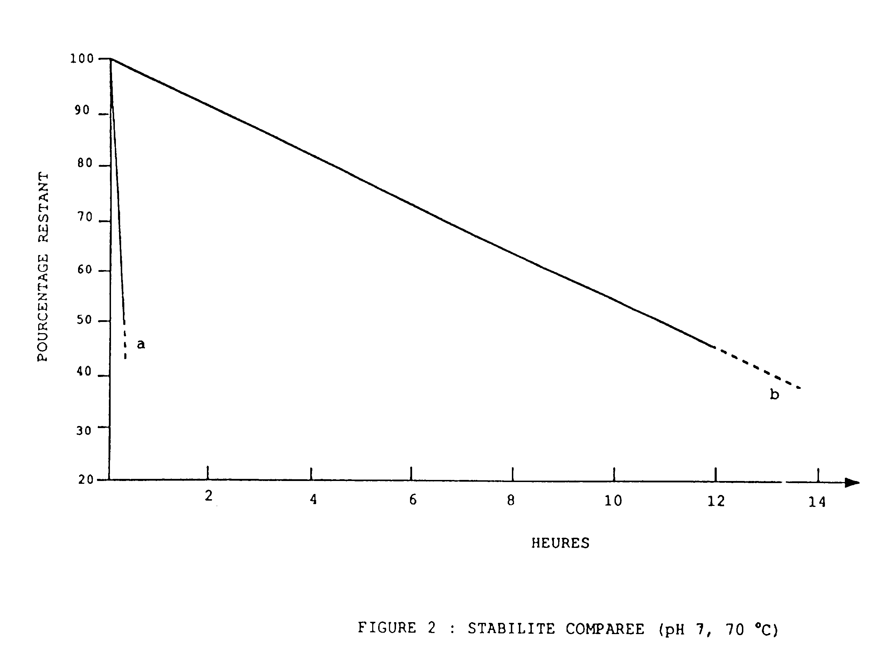

Dans la figure 2 annexée, est donné un diagramme comparatif de la courbe de stabilité de l'aspartame (courbe a) par rapport au composé de l'invention (courbe b) à pH 7; le composé de l'invention présente une demi-vie d'environ 11 heures, alors que la demi-vie de l'aspartame dans les mêmes conditions n'est que de 10 minutes, ce qui correspond, pour le composé selon l'invention, à une stabilité 66 fois plus élevée que celle de l'aspartame.

## Revendications

1. Composé édulcorant caractérisé en ce qu'il s'agit du *N*-[*N*-(3,3-diméthylbutyl)-L-α-aspartyl]-L-hexahydrophénylalanine 1-méthyl ester de formule :

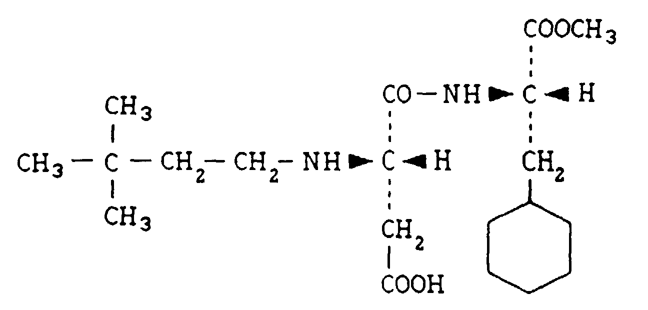

2. Procédé de préparation du *N*-[*N*-(3,3-diméthylbutyl)-L-α-aspartyl]-L-hexahydrophénylalanine 1-méthyl ester de formule : 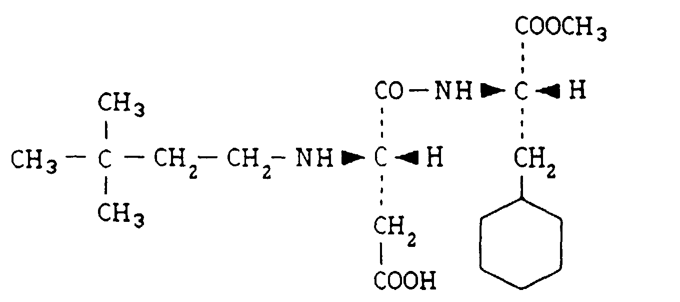 caractérisé en ce que l'on traite une solution d'aspartame et de 3,3-diméthylbutyraldéhyde à température ambiante par de l'hydrogène à une pression relative de 3 bars en présence d'un catalyseur à base de platine.

3. Procédé selon la revendication 2 caractérisé en ce que le catalyseur est le noir de platine ou l'oxyde de platine.

4. Procédé selon les revendications 2 et 3 caractérisé en ce que la solution d'aspartame et de 3,3-diméthylbutyraldéhyde est une solution hydroalcoolique de pH 4,5-5 obtenue par mélange d'une solution d'acide acétique 0,1 M et de méthanol.

5. Procédé selon les revendications 2 à 4 caractérisé en ce que la concentration d'aspartame dans le solvant hydroalcoolique est comprise entre 50 et 60 g/L et la concentration de 3,3-diméthylbutyraldéhyde est comprise entre 20 et 30 g/L.

6. Procédé selon les revendications 2 à 5 caractérisé en ce que le produit formé est purifié par précipitation et filtration, après élimination sous vide de la partie alcoolique du solvant.

## Patentansprüche

1. Süßstoff-Verbindung, dadurch gekennzeichnet, daß es sich handelt um N-[N-(3,3-Dimethylbutyl)-L-α-aspartyl]-L-hexahydrophenylalanin-1-methylester der Formel:

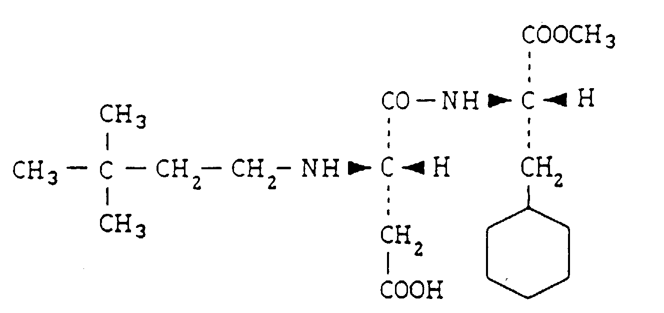

2. Verfahren zur Herstellung von N-[N-(3,3-Dimethylbutyl)-L-α-aspartyl]-L-hexahydrophenylalanin-1-methylester der Formel: 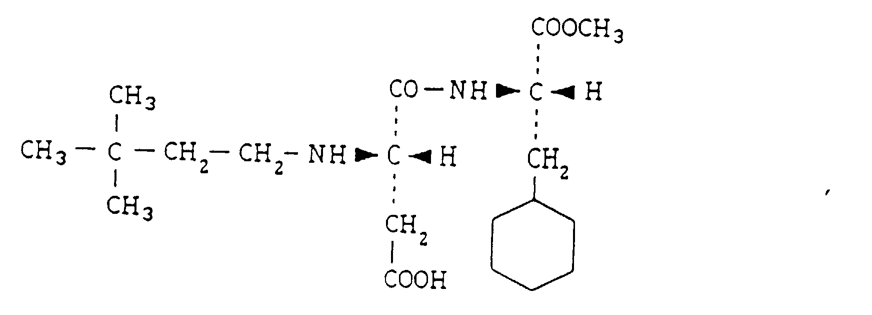 dadurch gekennzeichnet, daß eine Lösung von Aspartam und von 3,3-Dimethylbutyraldehyd bei Umgebungstemperatur mit Wasserstoff bei einem relativen Druck von 3 bar in Anwesenheit eines Katalysators auf der Basis von Platin behandelt wird.

3. Verfahren nach Anspruch 2, dadurch gekennzeichnet, daß der Katalysator Platinschwarz oder Platinoxid ist.

4. Verfahren nach den Ansprüchen 2 und 3, dadurch gekennzeichnet, daß die Lösung von Aspartam und von 3,3-Dimethylbutyraldehyd eine hydroalkoholische Lösung mit einem pH von 4,5 bis 5 ist, die durch Mischen einer Lösung von 0,1 M Essigsäure und Methanol erhalten wird.

5. Verfahren nach den Ansprüchen 2 bis 4, dadurch gekennzeichnet, daß die Aspartam-Konzentration im hydroalkoholischen Lösungsmittel zwischen 50 und 60 g/l liegt, und die 3,3-Dimethylbutyraldehyd-Konzentration zwischen 20 und 30 g/l liegt.

6. Verfahren nach den Ansprüchen 2 bis 5, dadurch gekennzeichnet, daß das gebildete Produkt durch Ausfällung und Filtration gereinigt wird, nachdem der Alkohol-Teil des Lösungsmittels im Vakuum eliminiert wurde.

## Claims

1. A sweetening compound characterised in that it is *N*-[*N*-(3,3-dimethylbutyl)-L-α-aspartyl]-L-hexahydrophenylalanine 1-methyl ester of formula:

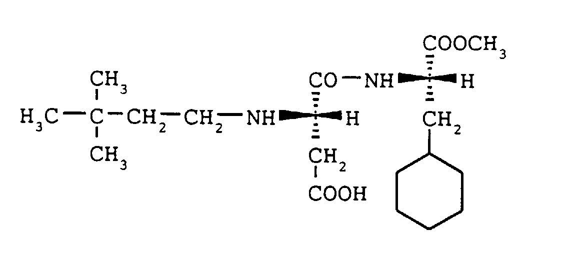

2. A method of preparation of *N*-[*N*-(3,3-dimethylbutyl)-L-α-aspartyl]-L-hexahydrophenylalanine 1-methyl ester of formula: 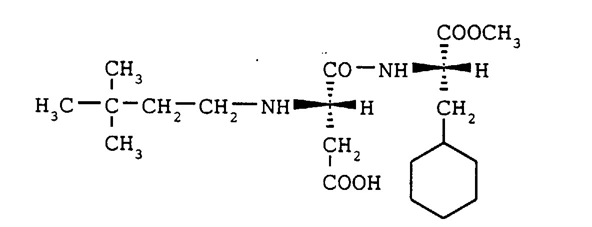 characterised in that a solution of aspartame and 3,3-dimethylbutyraldehyde at room temperature is treated with hydrogen at a relative pressure of 3 bars in the presence of a platinum-based catalyst.

3. The method according to claim 2, characterised in that the catalyst is platinum black or platinum oxide.

4. The method according to claims 2 and 3, characterised in that the solution of aspartame and 3,3-dimethylbutyraldehyde is an aqueous/alcohol solution of pH 4.5-5 obtained by mixing a 0.1M solution of acetic acid and methanol.

5. The method according to claims 2 to 4, characterised in that the concentration of aspartame in the aqueous/alcohol solution is between 50 and 60 g/l and the concentration of 3,3-dimethylbutyraldehyde is between 20 and 30 g/l.

6. The method according to claims 2 to 5, characterised in that the product formed is purified by precipitation and filtration, after the evaporation *in vacuo* of the alcohol part of the solvent.
